Europäisches Patentamt

European Patent Office

Office européen des brevets

(11) Publication number: **0 528 148 A1**

## EUROPEAN PATENT APPLICATION

(21) Application number: **92111064.9**

(22) Date of filing: **30.06.92**

(51) Int. Cl.5: **C12P 1/06, C12N 1/20,**
**//A61K35/74,A01N47/44,**
**(C12P1/06,C12R1:465),**
**(C12N1/20,C12R1:465)**

(30) Priority: **03.07.91 EP 91111039**

(43) Date of publication of application:
**24.02.93 Bulletin 93/08**

(84) Designated Contracting States:
**AT BE CH DE DK ES FR GB GR IT LI LU NL PT**
**SE**

(71) Applicant: **HOECHST AKTIENGESELLSCHAFT**
**Postfach 80 03 20**
**W-6230 Frankfurt am Main 80(DE)**

(72) Inventor: **Nadkarni, Suresh, Rudra, Dr.**
**5, Yagtneshwar Kasturba Marg Mulund**
**IN-Bombay 400 080(IN)**
Inventor: **Coutinho, Louis, Ernst, Linus, Dr. c/o**
**Hoechst India Ltd. L.B.S. Marg Mulund**
**IN-Bombay 400 080(IN)**
Inventor: **DE, Sibendu, Dr. c/o**
**Hoechst India Ltd. L.B.S. Marg Mulund**
**IN-Bombay 400 080(IN)**
Inventor: **Ganguli, Bimal, Naresh, Dr.**
**401-402"Vaikunth" 8th Road Sindhi Society**
**Chembur**
**IN-Bombay 400 080(IN)**
Inventor: **Blumbach, Jürgen, Dr.**
**Fasanenweg 8**
**W-6272 Niedernhausen(DE)**
Inventor: **Fehlhaber, Hans-Wolfram, Dr.**
**Thomas-Mann-Strasse 5a**
**W-6270 Idstein / Ts.(DE)**
Inventor: **Kogler, Herbert, Dr.**
**Breslauer Strasse 39**
**W-6233 Kelkheim / Ts.(DE)**
Inventor: **Sachse, Burkhard, Dr.**
**An der Ziegelei 30**
**W-6233 Kelkheim / Fischbach(DE)**
Inventor: **Braun, Peter, Dr.**
**Pfarrer-Dorn-Strasse 13**
**W-6500 Mainz 32(DE)**

(54) **Compounds M 871563 A and B, having a azalomycin like structure.**

(57) Compounds M 87 1563 A with molecular formula $C_{54}H_{92}N_3O_{17}$ and M 87 1563 B with molecular formula $C_{55}H_{94}N_3O_{17}$ have an antifungal activity.

The instant invention relates to new compounds M 871563 A and B, a process for their production as well as their use.

On the basis of spectroscopic evidence, these compounds may be classified as macrocyclic lactone antibiotics. Macrocyclic lactone antibiotics are described in the CRC Handbook of antibiotics, Vol. II, authored by J. Berdy (1980).

Among all the antibiotics, M 87 1563 A and M 87 1563 B are most similar to the Azalomycin antibiotics described by M. Arai in J. Antibiotics. Ser. A, 13, 46, 51 (1960); M. Arai, Arzneim. Forsch., 18, 1396 (1968); M. Arai and K. Hamono, J. Antibiotics, 23, 107 (1970). The structures of these antibiotics along with their physicochemical properties are described in the following reports: M. Namikoshi, K. Saskai, Y. Koiso, S. Iwasaki, K. Fukushima, S. Mosoe & S. Okuda, Chem. Pharm. Bull 30(5) 1653-57 (1982) and by the same authors in Chem. Pharm. Bull (30(5) 1658-1688 (1982); Chem. Pharm. 30(5) 1660-1673 (1982); Chem. Pharm. Bull. 30(11) 4006-4014 (1982).

However, from all the data available as well as from a direct comparison of the authentic sample of the Azalomycin complex on HPLC and other comparative methods, the compounds described herein differ from the known Azalomycins as well as from any other antibiotic described in the quoted literature. M 87 1563 A and M 87 1563 B have the molecular formulas $C_{54}CH_{92}N_3O_{17}$ and $C_{55}H_{94}N_3O_{77}$ respectively. An on-line Chemical Abstract search performed with the search keys of molecular formula and molecular weight shows that M 87 1563 A showed no similar compound. M 87 1563 B showed it to be corresponding to Azalomycin $F_3a$.

A comparison of the $^1H$ NMR, $^{13}C$ NMR and HPLC behavior clearly shows that M 87 1563 B is also different from any of the known Azalomycins including Azalomycin $F_3a$.

According to the present invention there is also provided a process for the production of the two new compounds M 87 1563 A and M 87 1563 B. The said process comprises cultivation of Streptomyces species culture No. Y-85,21242, its mutants and variants, under aerobic conditions in a nutrient medium containing sources of carbon and nitrogen, nutrient inorganic salts and trace elements and isolation and purification of the said compounds from the culture broth. Culture No. Y-85,21242 has been deposited under the conditions of the treaty of Budapest on May 21, 1991 with Deutsche Sammlung von Mikroorganismen, Braunschweig and has been designated No. DSM 6542.

The carbon sources for cultivation may be e.g. starch, glucose, sucrose or molasses. The preferred carbon source is glucose. The sources of nitrogen may be for instance soybean meal, peanut meal, yeast extract, beef extract, malt extract, corn steep liquor, peptone or casamino acids. The preferred nitrogen sources are soybean meal and peptone. Nutrient inorganic salts may be for instance sodium chloride, sodium hydrogen phosphate, potassium hydrogen phosphate, calcium chloride, calcium carbonate or magnesium sulphate. Trace elements could be salts of iron, manganese, copper, zinc, cobalt or such other heavy metals.

Cultivation of culture No. Y-85,21242 may be carried out at temperatures between 26 and 30°C and pH between 6.0 and 8.0. Preferably culture No. Y-85,21242 is cultivated at 28°C (± 1°C) and pH 6.8-7.0.

The fermentation is carried out for 60 to 80 hours when optimal yield of the compounds of the present invention is obtained. Fermentation is preferably carried out for 72 hours under submerged condition in shake flasks and for 70 hours in laboratory fermenters. If desired, Desmophen® (Polyols, Bayer AG, Leverkusen) may be used as an antifoam agent in the fermenters. The progress of fermentation and formation of Azalomycins of the present invention can be detected by measuring the bioactivity of the culture broth against Aspergillus niger or Neurospora crassa strains by the known agar plate diffusion assay method. The preferred test culture is Neurospora crassa.

In the resulting culture broth, the Azalomycins are present both in the culture filtrate as well as in the mycelium. The Azalomycins can be recovered from the mycelium by extraction with solvents such as methanol, ethanol, propanol. The preferred solvent for extraction is methanol. The Azalomycins from the culture filtrate can be extracted from the culture filtrate by one of the several known methods such as hydrophobic interaction chromatography on Diaion® HP-20 followed by desorption of the compound from the resin with suitable organic solvents such as methanol acetone, acetonitrile and an aqueous combination of these solvents. The preferred solvent for elution is aqueous methanol. The extracts obtained from the mycelium may be combined with the active eluates obtained from the desorption of the Diaion HP-20 column used for the culture filtrate or they may be processed independently. The preferred method is to combine these extracts and eluates and to concentrate the mixture under reduced pressure distillation to remove all the solvent or the major part of it.

The resulting concentrate or crude redissolved in methanol can be obtained as a powder by precipitation with a solvent such as acetone to give a crude mixture of the Azalomycin complex. The crude mixture thus obtained is an off-white coloured powder. Further purification can now be achieved by chromatography on substances such as silica gel, modified silica gels, cellulose or Sephadex® LH-20, G-10 or by crystallization from various solvents or by counter-current chromatography techniques using instruments such as Droplet Counter Current Chromatograph or Ito Coil or reprecipitation of the compound from a solution of the compound by solvents in which the compound is insoluble such as acetone, acetonitrile etc. The preferred method is a preliminary purification of the crude via chromatography. Chromatography on silica gel may be carried out by using solvents such as methanol, n-butanol, sec. butanol, tert. butanol, or aqueous combinations of these solvents. Chromatography on modified silica gels may be carried out on substrates such as octadecyldimethyl silyl silica (RP-18), using solvents such as methanol, acetonitrile, or aqueous combinations of these solvents which may also be buffered at various pHs using buffering agents such as salts like $Na_2HPO_4$, $NaH_2PO_4$ or reagents such as triethylamine and phosphoric acid the relative concentrations of which may be adjusted to give the desired pH.

The preferred method is successive series of chromatographic steps beginning with RP-18 using acetonitrile and water as eluents with the amount of acetonitrile being increased in a stepwise fashion. Fractions which are enriched in the desired component are then pooled and concentrated under reduced pressure and rechromatographed on RP-18 using as eluent a combination of acetonitrile, water, triethylamine and phosphoric acid, with the percentage of acetonitrile being increased in a stepwise manner whilst pH is maintained at about 7.0. Pure fractions thus obtained can be desalted by a variety of known methods such as readsorption of the eluates, after concentration under reduced pressure and dilution with water, onto substrates such as XAD-2, HP-20, RP-18, charcoal etc. or on gels such as Sephadex LH-20, G-10, G-25 etc. The preferred method is adsorption on RP-18 followed by desorption with methanol after washing with water to ensure the complete removal of all salts to give pure compounds M 87 1563 A and M 87 1563 B.

Further aspects of the instant invention are pharmaceuticals containing an active amount of compounds M 87 1563 A and/or B as well as the use of said compounds as an antifungal antibiotic.

The respective pharmaceuticals can optionally contain additives and/or excipients known in the art and can be prepared in a manner known per se.

Furthermore, the compounds according to the instant invention are distinguished by an excellent fungicidal action. Fungal pathogens which have already penetrated the plant tissue can be successfully controlled. This is particularly important and advantageous in the case of those fungal diseases where control with other fungicides, after infection has taken place, is no longer effective. The range of action of the claimed compounds embraces a multitude of various economically important phytopathogenic fungi such as, for example, Pyricularia oryzae, Venturia inaequalis, Cercospora beticola, Erysiphicaceae (powdery mildew species), Fusarium- und Leptosphaeria-species, Plasmopara viticola, Phytophthora infestans, Pseudoperonospora cubensis, various rusts, BCM- and/or Dicarboximide-sensitive and -resistant Botrytis cinerea strains, Sclerotinia sclerotiorum, BCM-resistant Pseudocercosporella herpotrichoides-strains and Pellicularia sasakii.

In addition, the compound according to the invention are also suitable for use in industrial sectors, for example as wood protection agents and as preservatives in paints.

The invention also relates to agents which contain the compounds of the formula I in addition to suitable formulation auxiliaries.

The agents according to the invention in general contain the active substances of the formula I in general in amounts of 1 to 95 % by weight.

They can be formulated in many ways, depending on the biological and/or physicochemical parameters. The following formulations are therefore possible: wettable powders (WP), emulsifiable concentrates (EC), aqueous solutions (SC), emulsions, sprayable solutions, oil- or water-based dispersions (SC), suspoemulsions (SC), dusts (DP), seed treatment agents, granules in the form of microgranules, spray granules, coated granules and adsorption granules, water-dispersible granules (WG), ULV formulations, microcapsules, waxes or baits.

These individual formulation types are known in principle and are described, for example, in: Winnacker-Küchler, "Chemische Technologie [Chemical Technology]", Volume 7, C. Hauser Verlag, Munich, 4th Ed., 1986; van Valkenburg, "Pesticides Formulations", Marcel Dekker N.Y., 2nd Ed. 1972-73; K. Martens, "Spray Drying Handbook", 3rd Ed. 1979, G. Goodwin Ltd. London.

The formulatjion auxiliaries required, such as inert materials, surfactants, solvents and other additives, are likewise known and are described, for example, in: Watkins, "Handbook of Insecticides Dust Diluents and Carriers", 2nd Ed., Darland Books, Caldwell N.Y.; H. v. Olphen, "Introduction to Clay Colloid Chemistry", 2nd Ed., J. Wiley & Sons, N.Y.; Marschen, "Solvents Guide", 2nd Ed., Interscience, N.Y. 1950; Mc Cutcheon's "Detergents and Emulsifiers Annual", MC Publ. Corp. Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte" [Surfaceactive Ethylene Oxide Adducts]", Wiss. Verlagsgesell., Stuttgart 1976; Winnacker-Küchler, "Chemische Technologie [Chemical Technology]", Volume 7, C. Hauser Verlag Munich, 4th Ed. 1986.

Combinations with other pesticidally active substances, fertilizers and/or growth regulators may also prepared on the basis of these formulations, for example in the form of a readymix or as a tank mix.

Wettable powders are preparations which are uniformy dispersible in water and which, besides the active substance, also contain wetting agents, for example polyoxethylated alkylphenols, polyoxethylated fatty alcohols, alkylsulfonates or alkylphenolsulfoantes, and dispersing agents, for example sodium ligninsulfonate, sodium 2,2'-dinaphthylmethane-6,6'-disulfonate, sodium dibutylnaphthalenesulfonate, or alternatively sodium oleymethyltaurinate, in addition to a diluent or inert substance. Emulsifiable concentrates are prepared by dissolving the active substance in an organic solvent, for example methanol, butanol, cyclohexanone, dimethylformamide, xylene and also higher-boiling aromatic compounds or hydrocarbons, with the addition of one or more emulsifiers. Examples of emulsifiers which can be used are: calcium salts of an alkylarylsulfonic acid, such as Ca dodecylbenzenesulfonate, or non-ionic emulsifiers, such as fatty acid polyglycol esters, alkylaryl polyglycol ethers, fatty alcohol polyglycol ethers, propylene oxide/ethylene oxide sorbitan fatty acid esters, polyoxyethylene sorbitan fatty acid esters or polyoxyethylene sorbitol esters.

Dusting agents can be obtained by grinding the active substance with finely divided solid substances, for example talc or natural clays, such as kaolin, bentonite, pyrophyllite or diatomaceous earth. Granules can be produced either by spraying the active substance onto adsorptive, granulated inert material or by applying active substance concentrates onto the surface of carriers, such as sand, kaolinites or granulated inert material, by means of binders, for example polyvinyl alcohol, sodium polyacrylate or, alternatively, mineral oils. Suitable active substances can also be granulated in the manner which is conventional for the production of fertilizer granules, if desired in a mixture with fertilizers.

The concentration of active substance in wettable powders is, for example, about 10 to 90 % by weight, the remainder to 100 % by weight is composed of conventional formulation components. In the case of emulsifiable concentrates, the concentration of active substance can be about 5 to 80 % by weight. Formulations in the form of dusts usually containg 5 to 20 % by weight of active substance, sprayable solutions about 2 to 20 % by weight. In the case of granules, the active substance content depends partly on whether the active compound is liquid or solid and on which granulation auxiliaries, fillers etc. are used.

In addition, the active substance formulations mentioned contain, if appropriate, the adhesives, wetting agents, dispersing agents, emulsifiers, penetrants, solvents, fillers or carriers which are conventional in each case.

For use, the concentrates, present in commercially available form, are diluted, if appropriate, in a customary manner, for example using water in the case of wettable powders, emulsifiable concentrates, dispersions and in some cases, also in the case of microgranules. Preparations in the form of dusts or granulated preparations and also sprayable solutions are usually not further diluted with other inert substances before use.

The application rate required varies with the external conditions, such as, inter alia, temperature and humidity. It can vary within wide limits, for example between 0.005 and 10.0 kg/ha or more of active ingredient: preferably, however, it is between 0.01 and 5 kg/ha.

The active substances according to the invention can be used, in their commercially available formulations, either by themselves or in combination with other fungicides known from the literature.

Examples of fungicides which are known from the literature and which can be combined according to the invention with the compounds of the formula I, are the following products:

anilazine, benalaxyl, benodanil, benomyl, binapacryl, bitertanol, buthiobat, captafol, captan, carbendazim, carboxin, CGD-94240 F, chlobenzthiazone, chlorthalonik, copper compounds like Cu-oxi-chloride, oxine-Cu, Cu-oxide, cymoxanil, cyproconazole, cyprofuram, dichlofluanid, dichlomezin, diclobutrazol, diethofencarb, difluconazole, dimethirimol, dimethomorph, diniconazole, dinocap, dithianon, dodemorp, dodine, edifenfos, ethirimol, etridiazol, fenarimol, fenfuram, fenpiclonil, fenpropidin, fenpropimorph, fentinacetate, fentinhydroxide, fluaziram, fluobenzimine, fluorimide, flusilazole, flutolanil, flutriafol, folpet, fosetylaluminium, fuberidazole, furalaxyl, furmecyclox, guazatine, hexaconazole, imazalil, iprobenfos, iprodione, isoprothiolane, mancozeb, maneb, mepronil, metalaxyl, methasulfocarb. methfuroxam, myclobutanil, nabam, nitrothalidopropyl, nuarimol, ofurace, oxadixyl, oxycarboxin, penconazol, pencycuron, PP 969, probenazole, probineb, prochloraz, procymidon, propamocarb, propiconazol, prothiocarb, pyracarbolid, pyrifenox, pyroquilon, rabenzazole, sulfur, tebuconazle, thiabendazole, thiofanatemethyl, thiram, tolclofosmethyl, tolylfluanid, triadimefon, triadimenol, tricyclazole, tridemorph, triflumizol, triforine, vinchlozolin, zineb, sodiumdodecylsulfonate, sodium-dodecysulfate, sodium-C13/C15-alcoholethersulfonate, sodium-cetostearylphosphateester, dioctyl-sodium-sulfosuccinate, sodium-isopropylnaphthalenesulfonate, sodium-methylenebisnaphtalenesulfonate, cetyl-trimethyl-ammonium-chloride, salts of long-chain primary, secondary or tertiary amines alky-propyleneamine, lauryl-pyrimidiniumbromide, ethoxilated quarternated lipidamines, alky-dimethyl-benzyl-ammonium-chloride and 1-hydroxyethyl-2-alkyl-imidazoline.

The abovementioned combination components represent known active substances, most of which are described in CH.R. Worthing, U.S.B. Walker, The Pesticide Manual, 7th Ed. (1983), British Crop Protection Council.

Moreover, the active substances according to the invention, in particular those of the examples mentioned, can be present in their commercially available formulations and in the use form prepared from these formulations in a mixture with other active substances, such as insecticides, attractants, sterilants, acaricides, nematicides, fungicides, growth-regulating substances or herbicides. Examples of insecticides include, inter alia, phosphoric esters, carbamates, carboxylic esters, formamidines, tin compounds and substance prepared by microorganisms. Preferred mixture components are:

1. from the group of the phosphoric esters

azinophos-ethyl, azinophos-methyl, 1-(4-chlorophenyl-4-(O-ethyl, S-propyl)phosphoryloxypyrazole (TIA 230), chlorpyrifos, coumaphos, demeton, demeton-S-methyl, diazinon, dichlorvos, di-methoate, ethoprophos, etrimfos, fenitrothion, fenthion, heptenophos, parathion, parathion-methyl, phosalone, pirimiphos-ethyl, pirimiphos-methyl- profenofos, prothiofos, sulprofos, triazophos and trichlorphon.

2. from the group of the carbamates

aldicarb, bendiocarb, BPMC (2-(1-methylpropyl)phenyl-methyl-carbamate), butocarboxim, butoxicarboxim, carbaryl, carbofuran, carbosulfan, cloethocarb, isoprocarb. methomyl, oxamyl, pirimicarb, promecarb, propoxur and thiodicarb.

3. from the group of the carboxylic esters

allethrin, alphamethrin, bioallethrin, bioresmethrin, cycloprothrin, cyfluthrin, cyhalothrin, cypermethrin, deltamethrin, alpha-cyano-3-phenyl-2-methylbenzyl, 2,2-dimethyl-3-(2-chloro-2-trifluoromethylvinyl)-cyclopropanecarboxylate(FMC 54800), fenpropathrin, fenfluthrin, fenvalerate, flucythrinate, flumethrin, fluvalinate, permethrin, resmethrin, tralomethrin.

4. from the group of the formamidines

amitraz and chlordimeform

5. from the group of the tin compounds

azocyclotin, cyhexatin and fenbutatin oxide

6. others

abamectin, Bacillus thuringiensis, bensultap, binapacryl, bromopropylate, buprofecin, camphechlor, cartap, chlorbenzilate, chlorfluazuron, 2-(4-chlorophenyl)-4,5-diphenylthiophene (UBI-T 930), chlofentenzine, 2-naphthylmethyl cyclopropanecarboxylate (Ro 12-0470), cyromacin, DDT, dicofol, N-(3,5-dichloro-4-(1,1,2,2-tetrafluoroethoxy)phenylamino)carbonyl)-2,6-difluorobenzamide (XRD 473), diflubenzuron, N-(2,3-dihydro-3-methyl-1,3-thiazol-2-ylidene)2,4-xylidine, dinobuton, dinocap, endosulfan, fenoxacarb, fenthiocarb, flubenzimine, flufenoxuron, gamm-HCH, hexythiazox, hydramethylnon (AC 217 300), ivermectin, 2-nitromethyl-4,5-dihydro-6H-thiazine (SD 52618), 2-nitromethyl-3,4-dihydrothiazole (SD 35651)2-nitromethylene-1,3-thiazinan-3-ylcarbamaldehyde (WL 108 477), propargite, teflubenzuron, tetradifon, tetrasul, thiocyclam, triflumaron, and nuclear polyhedrosis and granulosis viruses.

The active substance content of the use forms prepared from the commercially available formulations can vary within broad ranges. The active substance concentration of the use forms can be from 0.0001 to 100 % by weight of active substance, preferably between 0.001 and 1 % by weight. Application is effected in a conventional fashion, matched to the use forms.

The following examples are illustrative of the present invention which is also further characterized by the content of the patent claims.

Example 1

Isolation of the culture Y-85,21242 from soil.
a) Composition of nutrient isolation medium

| | |
|---|---|
| Glucose | 35.0 g |
| Dextrin | 10.0 g |
| Beef extract | 3.0 g |
| Peptone | 3.0 g |
| Yeast extract | 3.0 g |
| Soytone | 10.0 g |
| NaCl | 2.0 g |
| $K_2HPO_4$ | 0.5 g |
| $MgSO_4 \bullet 7H_2O$ | 0.2 g |
| $(NH_4)_2SO_4$ | 1.0 g |
| $COCl_2 \bullet 6H_2O$ | 0.001 g |
| Trace salt solution[*] | 1 ml |
| Demineralized water | 1000 ml |
| pH 7.3 | |

* Trace salts solution

| | |
|---|---|
| $CuSO_4 \bullet 5H_2O$ | 7.0 g |
| $FeSO_4 \bullet 7H_2O$ | 1.0 g |
| $MnCl_2 \bullet 4H_2O$ | 8.0 g |
| $ZnSO_4 \bullet 7H_2O$ | 2.0 g |
| Demineralized water | 1000 ml |

b) Soil Plating and Isolation:

10 g of soil collected from Dang forest were added to 90 ml of sterilized demineralized water in a 250 ml Erlenmeyer flask which was shaken for 2 hours on a rotary shaker (220 rpm). The above soil suspension was then serially diluted in steps of 10 up to $10^{-5}$. From the last dilution, 1 ml of suspension was placed at the center of a sterile glass petri plate (15 cm diameter) to which was then poured approximately 50 ml of the above isolation medium supplemented with 25 mcg/ml of amphotericin B and cooled to 45°C and the plate swirled thoroughly. The mixture of soil suspension and medium was allowed to settle and incubated at 28°C (± 1°C) for 7 days. The petri plate was periodically observed and the culture No. Y-85,21242 was isolated from amongst the growing microorganisms.

Example 2

Maintenance of the culture Y-85,21242

Composition of maintenance medium

Culture No. Y-85,21242 was maintained on nutrient agar medium with the following composition.

| Corn Starch | 10.0 g |
|---|---|
| Casein | 1.0 g |
| peptone | 1.0 g |
| Yeast extract | 1.0 g |
| $K_2HPO_4$ | 0,5 g |
| DM water | 1000 ml |
| pH 7.5 | |

After dissolving the ingredients thoroughly by heating, it was distributed in test tubes and then sterilized at 121°C for 20 minutes. The test tubes were cooled and allowed to solidify in a slanting position. The agar slants were streaked with the growth of the culture No. Y-85-21242 by a wire loop and incubated for 4 days at 28°C (± 1°C) when a good growth was observed. The well grown cultures were stored in the refrigerator at +8°C.

Example 3

Fermentation of culture Y-85,21242 in shake flasks

Composition of seed culture medium

| Glucose | 10.0 g |
|---|---|
| Sucrose | 20.0 g |
| Soybean meal | 15.0 g |
| Tryptone | 5.0 g |
| Yeast extract | 5.0 g |
| $K_2HPO_4$ | 0.2 g |
| DM water | 1 litre |
| pH 6.8 | |

The above seed culture medium was distributed in 60 ml amounts in 500 ml Erlenmeyer flasks and autoclaved at 121°C for 20 minutes. The flasks were cooled and each was inoculated with a loopful of the above mentioned well grown culture of Example 2 and shaken for 72 hours at 220 rpm at 28°C (± 1°C). This was used as seed culture for inoculating the production flasks as discussed below.

Production of the compounds in shake flasks

Composition of the production medium:

| Glucose | 25.0 g |
| Soybean meal | 10.0 g |
| Peptone | 5.0 g |
| $CaCO_3$ | 0.2 g |
| $COCl_2 \cdot 6H_2O$ | 0.001 g |
| DM water | 1 litre |
| pH 6.8 | |

The production medium was distributed in 60 ml amounts in 500 ml Erlenmeyer flasks and autoclaved at 121°C for 20 minutes. The flasks were cooled and then inoculated with the above mentioned seed culture (2 % v/v). The fermentation was carried out on a rotary shaker at 220 rpm and at a temperature of 28°C (± 1°C) for 72 hours.

The production of the compounds was determined by the bioactivity tested against Neurospora crassa using the well diffusion method in a known manner. After harvesting, the culture broth (pH 6) was centrifuged and M 87 1563 A and B were isolated from the culture filtrate and from mycelium and purified as described herein below.

Example 4

Cultivation of the culture No. Y-85,21242 in fermenters

Preparation of seed culture

Stage 1: The seed culture medium described in Example 3 was distributed in 60 ml amounts in 500 ml Erlenmeyer flasks and autoclaved at 121°C for 20 minutes, cooled and inoculated with a loopful of the well grown culture of Example 2. The flasks were incubated at 28°C (± 1°C) for 72 hours at 220 rpm on a rotary shaker. This growth was used to inoculate the stage 2 seed culture medium.

Stage 2: Ten liters of seed culture medium described in Stage 1 were taken in 15 litre fermenter and sterilized for 36 minutes at 121°C. On cooling, the fermenter was inoculated with seed culture from Stage 1(10 % v/v) and the fermenter was run with the following parameters:

| Temperature: | 28°C (± 0.1°C) |
| Agitation: | 180 r.p.m. |
| Aeration: | 8 LPM |
| Period: | 24 hours |

Large Scale Fermentation

110 litre of the production medium (same as Production medium mentioned in Example 3) in 150 litre fermenter with presterilization pH adjusted to 6.6 and with 0.04 % Desmophen® were sterilized in situ for 28 minutes at 121°C, and inoculated with the seed culture from stage 2(10 % v/v).

The fermenter was run with the following parameters:

| Temperature: | 28°C (± 0.5°C) |
| Agitation: | 100 r.p.m. |
| Aeration: | 60-70 liters per minute |
| Harvest time: | 69 hours |

The production of the compounds was determined by the activity profile tested against Neurospora crassa. The culture broth (pH 6.5) was centrifuged after harvesting to separate culture filtrate from mycelium. The compounds were isolated and purified from both the culture filtrate and the mycelium as mentioned herein below.

Example 5

Approximately 87 liters of the harvested broth were separated from the mycelium by centrifugation.

The resulting filtrate (pH 6-6.5) was passed through a column (4.5 liters) of Diaion HP-20. The column was washed with 30 liters of demineralized water. Elution was then carried out using 20 liters each, of a mixture of 40:60, 50:50, 60:40, 70:30, 80:20 proportions (v/v) of methanol:water. The resulting eluates were collected in 5 litre fractions. The Azalomycins eluted out (as monitored by bioactivity against Neurospora crassa) in the eluates containing mixtures of 70:30 and 80:20 proportion of methanol:water.

The mycelium (5 kg) was extracted with 20 liters of methanol and the extract was separated by filtration. The process was repeated once again.

The methanol extracts of the mycelium were pooled with the bioactive fractions of the Diaion HP-20 column to give a total volume of 75 liters and concentrated under reduced pressure to remove most of the solvent. To the residual concentrate (3 liters) was added acetone (8 liters) to precipitate a crude mixture of the Azalomycins as an off-white powder (105 g).

The crude mixture (5 g) was subjected to medium pressure liquid chromatography (MPLC) over 500 g of RP-18 (30 $\mu$) packed in a glass column. The column was developed initially in water at a flow rate of 20 ml min$^{-1}$ and subsequently eluted with 5 liters batches of solvent mixture, each comprising of 10:90, 20:80, 30:70, 40:60, 50:50, 60:40 ratio (v/v) of acetonitrile and water. The resulting eluates were collected in fractions each of 500 ml size and tested for bioactivity against N. crassa. The Azalomycins were seen to elute out in the fraction containing 50:50 and 60:40 ratio of acetonitrile and water. The active eluates then obtained were pooled and concentrated under reduced pressure to give a mixture of the Azalomycins as a white powder (2 g). Further purification of this mixture (1 g) was carried out by MPLC on 200 g of RP-18 (30 $\mu$) packed in a glass column. The column was developed at a flow rate of 8 ml min$^{-1}$ with the following eluents: (acetonitrile + water) + triethylamine (0.1 %) + phosphoric acid (to adjust to pH 7). The ratio of acetonitrile in water was increased in stages from 10:90, 20:80, 25:75, 30:70, 35:65, 40:60, 45:55 for every 5 liters of solvent. The fractions (each of 250 ml size) were studied on high performance liquid chromatography (HPLC) using conditions which are described in detail under the section on physico-chemical properties.

On the basis of HPLC data, fractions containing only M 87 1563 A were pooled and concentrated under reduced pressure to a volume of approximately 50 ml. A concentrate of M 87 1563 B was obtained in a similar manner.

M 87 1563 A was obtained in a pure form by the following method. To the concentrate (50 ml) was added a further 50 ml of demineralized water. This solution was charged onto a RP-18 glass column (30 $\mu$) (60 g) under MPLC conditions. The column was then washed with demineralized water (2 liters) and further eluted with methanol at a flow rate of 4 ml/min$^{-1}$ to yield eluates which were collected in fraction of volume 15 ml each. The bioactive fractions were studied on HPLC in the same manner as described previously and the suitable fractions were lyophilized to give M 87 1563 A (300 mg). M 87 1563 B (120 mg) was obtained in a similar manner.

Physico-chemical properties

|  | M 87 1563 A | M 87 1563 B |
|---|---|---|
| Appearance: | white powder | white powder |
| Solubility | MeOH, DMSO | MeOH, DMSO |
| High res. FAB MS: Mz: | $1053.642 \pm 0.003$ (M+H$^+$) | $1067.657 \pm 0.003$ (M+H$^+$) |
| Molecular formula: | $C_{54}H_{92}N_3O_{17}$ | $C_{55}H_{94}N_3O_{17}$ |
| UV: | 238, 260 Sh | 238, 260 (Sh) |
| HPLC: | Column RP-18 (Lichrospher®, | |

60 Rp Select B) (125 x 4 mm) (5 $\mu$).

Detection: UV at 240 nm

Solvent System : Water:Acetonitrile (65:35) + 0.1 % Triethylamine + Phosphoric Acid to adjust to desired pH.

$^{13}$C-NMR data:       see Table 1

(40 mg/ml in CD$_3$OD

100.62 MHz)

Table 1       M 87 1653

| A (m/z = 1053) | B (m/z = 1067) |
|---|---|
| 10.43 | 10.43 |
|  | 12.95 |
| 13.39 |  |
| 14.10 | 14.26 |
| 14.94 | 14.81 |
| 16.94 | 16.88 |
| 17.55 | 17.82 |
| 27.94 | 27.82 |
|  | 28.34 |
| 29.75 | 29.78 |
| 30.52 | 30.20 |
| 30.52 | 30.52 |
| 33.46 | 33.53 |
| 33.53 | 33.53 |
| 34.39 | 34.08 |
| 35.08 | 34.99 |
| 39.36 | 39.36 |
| 40.75 | 40.46 |
| 40.91 | 40.91 |
| 41.18 | 41.29 |
| 41.85 | 41.85 |
| 41.85 | 41.95 |
| 41.85 | 41.95 |
| 44.08 | 44.19 |
| 44.46 | 44.35 |
| 44.54 | 44.54 |

| | |
|---|---|
| 44.56 | 44.56 |
| 46.39 | 46.33 |
| 56.06 | 56.06 |
| 65.48 | 65.57 |
| 65.64 | 65.57 |
| 66.32 | 66.22 |
| 69.66 | 69.66 |
| 70.62 | 70.77 |
| 72.31 | 72.13 |
| 72.41 | 72.64 |
| 74.31 | 74.31 |
| 74.88 | 75.05 |
| 75.52 | 75.52 |
| 77.28 | 77.60 |
| 80.36 | 80.62 |
| 99.73 | 99.84 |
| 125.06 | 125.45 |
| 120.72 | 120.63 |
| 130.03 | 130.25 |
| 128.43 | 128.37 |
| 130.10 | 130.25 |
| 132.60 | 132.52 |
| 136.15 | 136.26 |
| 139.82 | 139.85 |
| 146.64 | 146.64 |
| 147.34 | 147.34 |
| 158.61 | 158.19 |
| 169.89 | 169.93 |
| 171.58 | 171.64 |
| 174.97 | 174.99 |

| Retention Time (mins): | | |
|---|---|---|
| pH | M 87 1563 A | M 87 1563 B |
| 7.0 | 11.6 | 13.33 |
| 7.5 | 11.6 | 15.47 |
| 5.3 | 11.6 | 13.3 |
| 3.0 | 14.6 | 17.4 |

| Biological activity of M 87 1563 A and 87 1563 B | | |
|---|---|---|
| Test Organisms | MIC (mg/L) | |
| | M 87 1563 A | M 87 1563 B |
| Staphylococcus aureus 209 P | 100 | 100 |
| Escherichia coli 9632 | > 100 | > 100 |
| Candida albicans | 5.0 | 5.0 |
| Aspergillus niger | 5.0 | 5.0 |
| Fusarium culmorum 100 | 5.0 | 5.0 |
| Pyricularia oryzae 154 | 5.0 | 5.0 |
| Alternaria mali P 37 | 5.0 | 5.0 |
| Botrytis cinerea 47 | 5.0 | 5.0 |
| Botrytis cinerea 47 | 2.5 | 2.5 |
| Botrytis cinerea 57 | 2.5 | 2.5 |
| Pellicularia sasakii J03 | 5.0 | 5.0 |
| Leptosphaeria nodorum J02 | 5.0 | 5.0 |
| Pseudocercosporella herpotrichoides 008 | 5.0 | 5.0 |
| Neurospora crassa SGF 18 | 2.5 | 2.5 |

A. Formulation Examples

a) A dust is obtained by mixing 10 parts by weight of active substance and 90 parts by weight jof talc as inert material and comminuting the mixture in a hammer mill.

b) A wettable powder which is readily dispersible in water is obtained by mixing 25 parts by weight of active substance, 65 parts by weight of kaolincontaining quartz as inert material, 10 parts by weight of potassum ligningsulfonate and 1 part by weight of sodium oleoylmethyltaurinate as wetting and dispersing agent, and grinding the mixture in a pin disk mill.

c) A dispersion concentrate which is readily dispersible in water is prepared by mixing 40 parts by weight of active substance with 7 parts by weight of sulfosuccinic monoester, 2 parts by weight of a sodium salt of ligninsulfonic acid and 51 parts by weight of water and grinding the mixture in a ball mill to a fineness of below 5 microns.

d) A emulsifiable concentrate can be prepared from 15 parts by weight of active substance, 75 parts by weight of cyclohexanone as solvent and 10 parts by weight of oxethylated nonylphenol (10 EO) as emulsifier.

e) Granules can be prepared from 2 to 15 parts by weight of active substance and an inert carrier material for granules such as atttapulgite, granulated pumice and/or quarz sand. It is advantageous to use a suspension of the wettable powder from example b) with a solids content of 30 % which is sprayes onto the surface of attapulgite granules, and these are dried and intimately mixed. The proportion by weight of the wettable powder is about 5 % here, and that of the inert carrier material about 95 % of the finished granules.

B Biological examples

Example B1

Wheat plants of the variety of "Diplomat", about 4 weeks old, were uniformly wetted to runoff point with an aqueous, methanol-containing suspension of the claimed compound.

After the spray coating had dried on, the plants were inoculated with an aqueous spore suspension of Pseudocercosporella herptotrichoides and incubated at 95-100 % relative atmospheric humidity and 16°C. Evaluation of the tests was carried out 4 weeks after inoculation. The degree of effectiveness of the test substance was assessed as a percentage of the untreated infected control and can be seen from table 2.

Table 2

| Compound according to example B 1 | Degree of effectiveness with Pseudocercosporella herpotr. in % at mg of active substance per liter of spray liquor | | |
|---|---|---|---|
| | 600 | 300 | 150 |
| M-871563 | 100 | 100 | 100 |
| untreated | 0 | | |

Example B2

Broad beans of the varieties of "Herz Freya" or "Diana" about 14 days old were uniformly wetted to runoff point with an aqueous, methanol containing suspension of the claimed compound. After the spray coating had dried on, the plants were inoculated with a spore suspension (1,5 million spores/ml) of Botrytis cinerea (BCM-resistant-strain). The plants were cultivated further in a climatic chamber at 20-22°C and about 99 % relative atmospheric humidity. The infection of the plants is manifested by the formation or black spots on the leaves and stalks. Evaluation of the tests was carried out 1 week after inoculation.

The degree of effectiveness of the test substance was assessed as a percentage of the untreated infected control and is reproduced in table 3.

Table 3

| Compound according to example B2 | Degree of effectiveness with Botrytis cinerea in % at mg of active substance per liter of spray liquor | | | |
|---|---|---|---|---|
| | 150 | 75 | 40 | 20 |
| | 100 | 100 | 100 | 100 |
| untreated, infected plants | 0 | | | |

Example B3

Tomato plants variety "Rheinlands Ruhm" in the 3-4 leaf stage were uniformly wetted to runoff point with an aqueous, methanol containing suspension of the claimed compound.

After the coating had dried on, the plants were inoculated with a zoosporangia suspension of Phytophthora infestans and kept in a controlled-environment chamber with optimum infection conditions for 2 days. The plants were then grown further in the greenhouse until the symptoms became apparent.

The plants were scored for disease about 1 week after the inoculation. The degree of effectiveness of the test substance was scored as a percentage relative to the untreated control and can be seen from table 4.

14

Table 4

| Compound according to example B3 | Degree of effectiveness with Phytophthora infestans in % at mg of active substance per liter of spray liquor | | |
|---|---|---|---|
| | 600 | 300 | 150 |
| M-871563 | 100 | 100 | 97 |
| untreated, infected plants | 0 | | |

**Claims**

1. Compounds M 87 1563 A with molecular formula $C_{54}H_{92}N_3O_{17}$ and M 87 1563 B with molecular formula $C_{55}H_{94}N_3O_{17}$.

2. The compounds M 87 1563 A and B as claimed in claim 1, obtainable by cultivation of culture Y-85,21242 and subsequent isolation and purification.

3. A process for the production of the compounds as claimed in claims 1 or 2, wherein culture Y-85,21242 is cultivated in an aqueous nutrient medium containing inorganic salts and trace elements and said compounds are isolated from the culture both.

4. A process as claimed in claim 3, wherein cultivation is carried out at a temperature between 26 and 30°C and pH between 6 and 8.

5. A process as claimed in claims 3 or 4, wherein cultivation is carried out at a temperature of 28°C (± 1°C) and pH of 6.8 to 7.0.

6. A process as claimed in one or more of claims 3 to 5, wherein cultivation is carried out under aerobic conditions as a submerged cultivation.

7. A pharmaceutical containing an effective amount M 87 1563 A and/or B as claimed in claim 1.

8. The use of compounds as claimed in claim 1 for the preparation of a pharmaceutical.

9. The use of compounds as claimed in claim 1 for the preparation of a pharmaceutical having an antifungal activity.

10. A fungicidal agent, containing an effective amount of a compound as claimed in claim 1.

11. A fungicidal agent as claimed in claim 10, which contains 1 to 95 % by weight of a compound as claimed in claim 1 and 5 to 99 % by weight of common formulation auxiliaries.

12. A method of controlling harmful fungi, which comprises applying an effective amount of a compound of the formula I according to claim 1 to the plants, areas or substrates which are diseased with the fungi.

13. The use of a compound as claimed in claim 1 for controlling harmful fungi.

14. Streptomyces species Y-85,21242 (DSM 6542).

**Claims for the following Contracting States : ES, GR**

1. A process for the production of the compounds M 87 1563 A with molecular formula $C_{54}H_{92}N_3O_{17}$ and M 98 1563 B with molecular formula $C_{55}H_{94}N_3O_{17}$, wherein culture Y-85,21242 is cultivated in an aqueous nutrient medium containing inorganic salts and trace elements and said compounds are isolated from the culture broth.

**2.** A process as claimed in claim 1, wherein cultivation is carried out at a temperature between 26 and 30°C and pH between 6 and 8.

**3.** A process as claimed in claims 1 or 2, wherein cultivation is carried out at a temperature of 28°C (± 1°C) and pH of 6.8 to 7.0.

**4.** A process as claimed in one or more of claims 1 to 3, wherein cultivation is carried out under aerobic conditions as a submerged cultivation.

**5.** A pharmaceutical containing an effective amount of M 87 1563 A and/or B as claimed in claim 1.

**6.** The use of compounds as claimed in claim 1 for the preparation of a pharmaceutical.

**7.** The use of compounds as claimed in claim 1 for the preparation of a pharmaceutical having an antifungal activity.

**8.** A fungicidal agent, containing an effective amount of a compound as claimed in claim 1.

**9.** A fungicidal agent as claimed in claim 8, which contains 1 to 95 % by weight of a compound as claimed in claim 1 and 5 to 99 % by weight of common formulation auxiliaries.

**10.** A method of controlling harmful fungi, which comprises applying an effective amount of a compound according to claim 1 to the plants, areas or substrates which are diseased with the fungi.

**11.** The use of a compound as claimed in claim 1 for controlling harmful fungi.

**12.** Streptomyces species Y-85,21242 (DSM 6542)

16

European Patent Office

**PARTIAL EUROPEAN SEARCH REPORT**
which under Rule 45 of the European Patent Convention
shall he considered, for the purposes of subsequent
proceedings, as the European search report

Application Number

EP    92 11 1064

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| P,X | WO-A-9 204 457 (GRUPPO LEPETIT) 19 March 1992 The compounds GE1655B and GE1655C and their use | 1,2,7-13 | C12P1/06 C12N1/20 //(C12P1/06; C12R1:465) //(C12N1/20; C12R1:465) A61K35/74 A01N47/44 |
| D,A | CHEMICAL AND PHARMACEUTICAL BULLETIN. vol. 30, no. 11, 1982, TOKYO JP pages 4006 - 4014 S.IWASAKI C.S. 'STUDIES ON MACROCYCLIC LACTONE ANTIBIOTICS. V. THE STRUCTURES OF AZALOMYCINS F3A AND F5A.' * the whole document * | 1-14 | |

-----

**TECHNICAL FIELDS SEARCHED (Int. Cl. 5)**

C12P
C12N
C12R
A61K
A01N

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 09 OCTOBER 1992 | GROENENDIJK M.S.M. |

EPO FORM 1503 03.82 (P04E07)

EP 92 11 1064

-C-

Remark: Although claims 12 and 13
are partially  directed to a method of
treatment of the human/animal
body (Art. 52(4) EPC) the search
has been carried out and based on
the alleged effects of the
compound/composition